(19)

Europäisches Patentamt

European Patent Office

Office européen des brevets

(11) **EP 3 216 461 A1**

(12) **EUROPEAN PATENT APPLICATION**

(43) Date of publication:
**13.09.2017 Bulletin 2017/37**

(21) Application number: **16305253.3**

(22) Date of filing: **07.03.2016**

(51) Int Cl.:
**A61K 39/395** (2006.01)     **C07K 16/28** (2006.01)
**A61K 39/00** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Sanofi Biotechnology**
**75008 Paris (FR)**

• **REGENERON PHARMACEUTICALS, INC.**
**Tarrytown, NY 10591 (US)**

(72) Inventor: **The designation of the inventor has not yet been filed**

(74) Representative: **Catillon, Marie et al**
**Sanofi**
**Département Brevets**
**54, rue la Boétie**
**75008 Paris (FR)**

(54) **COMPOSITIONS AND METHODS FOR TREATING RHEUMATOID ARTHRITIS**

(57)    The present invention relates to the use of an anti-IL6 receptor antibody in monotherapy for treating rheumatoid arthritis and for improving the physical function and the quality of life of a subject suffering from rheumatoid arthritis.

EP 3 216 461 A1

SEQUENCE LISTING

```
<110>  SANOFI BIOTECHNOLOGY
       REGENERON PHARMACEUTICALS INC.

<120>  COMPOSITIONS AND METHODS FOR TREATING RHEUMATOID ARTHRITIS

<130>  FR2016-007

<160>  2

<170>  PatentIn version 3.5

<210>  1
<211>  116
<212>  PRT
<213>  Homo sapiens


<220>
<221>  MISC_FEATURE
<222>  (1)..(116)
<223>  heavy chain variable region of sarilumab

<400>  1

Glu Val Gln Leu Val Glu Ser Gly Gly Gly Leu Val Gln Pro Gly Arg
1               5                   10                  15


Ser Leu Arg Leu Ser Cys Ala Ala Ser Arg Phe Thr Phe Asp Asp Tyr
            20                  25                  30


Ala Met His Trp Val Arg Gln Ala Pro Gly Lys Gly Leu Glu Trp Val
            35                  40                  45


Ser Gly Ile Ser Trp Asn Ser Gly Arg Ile Gly Tyr Ala Asp Ser Val
        50                  55                  60


Lys Gly Arg Phe Thr Ile Ser Arg Asp Asn Ala Glu Asn Ser Leu Phe
65                  70                  75                  80


Leu Gln Met Asn Gly Leu Arg Ala Glu Asp Thr Ala Leu Tyr Tyr Cys
                85                  90                  95


Ala Lys Gly Arg Asp Ser Phe Asp Ile Trp Gly Gln Gly Thr Met Val
                100                 105                 110


Thr Val Ser Ser
                115


<210>  2
<211>  107
<212>  PRT
<213>  Homo sapiens
```

EP 3 216 461 A1

```
<220>
<221>  MISC_FEATURE
<222>  (1)..(107)
<223>  light chain variable region of sarilumab

<400>  2

Asp Ile Gln Met Thr Gln Ser Pro Ser Ser Val Ser Ala Ser Val Gly
1               5               10              15


Asp Arg Val Thr Ile Thr Cys Arg Ala Ser Gln Gly Ile Ser Ser Trp
        20              25              30


Leu Ala Trp Tyr Gln Gln Lys Pro Gly Lys Ala Pro Lys Leu Leu Ile
        35              40              45


Tyr Gly Ala Ser Ser Leu Glu Ser Gly Val Pro Ser Arg Phe Ser Gly
        50              55              60


Ser Gly Ser Gly Thr Asp Phe Thr Leu Thr Ile Ser Ser Leu Gln Pro
65              70              75              80


Glu Asp Phe Ala Ser Tyr Tyr Cys Gln Gln Ala Asn Ser Phe Pro Tyr
        85              90              95


Thr Phe Gly Gln Gly Thr Lys Leu Glu Ile Lys
        100             105
```

## Description

### FIELD OF THE INVENTION

The present invention relates to the field of rheumatoid arthritis. More specifically, the invention relates to methods of improving the physical function of subjects suffering from rheumatoid arthritis, methods of improving the health related quality of life of subjects suffering from rheumatoid arthritis, and methods of treating rheumatoid arthritis in subjects suffering from rheumatoid arthritis, comprising administering to the subject an anti-IL6 receptor antibody in monotherapy.

### BACKGROUND

It is estimated that approximately 0.5% to 1% of the adult population in North America and Europe is affected by rheumatoid arthritis (RA). RA affects women twice as often as men and the incidence is highest among women over 40 years of age.

RA is characterized by persistent synovitis and progressive destruction of cartilage and bone in multiple joints. The hallmark of the disease is a symmetric polyarthritis characteristically involving the small joints of the hands and feet. The inflammatory process can also target other organs, characteristically bone marrow (anemia), eye (scleritis, episcleritis), lung (interstitial pneumonitis, pleuritis), cardiac (pericarditis) and skin (nodules, leukocytoclastic vasculitis). Systemic inflammation is characterized by laboratory abnormalities, such as anemia, elevated erythrocyte sedimentation rate, fibrinogen and C-reactive protein (CRP) and by clinical symptoms of fatigue, weight loss, muscle atrophy in affected joint areas. The presence of polyclonal high-titer rheumatoid factors and anticyclic citrullinated peptide (anti-CCP) antibodies provides evidence of immune dysregulation. It has been estimated that 65% to 70% of RA patients have progressive disease that leads to joint destruction, disability and premature death.

In addition to improving the clinical symptoms of RA patients, there is a growing interest in improving the physical function and the health related quality of life of RA patients. Indeed, in addition to the usual instruments intended to assess health status of the patients (presence or absence of disease), physicians and clinicians developed new instruments to measure the physical function and quality of life of the patients, which are useful parameters for the physician to assess the overall response of his or her patient to a particular treatment. Quality of life for instance goes beyond the impairment/disability and handicap continuum by asking what patients' health status prevents them from doing and also about their emotional response to these restrictions. Quality of life also reflects the influences of the personal, social and economic resources that an individual has and the way in which these interact with health status (British Journal of Rheumatology 1997;36:884-888). The physical function assessment of RA patients typically takes into account the fine movements of the upper extremity, locomotor activities of the lower extremity, and activities that involve both the upper and lower extremities. These parameters are now widely used by the physicians, clinicians and regulatory agencies to compare the different treatment options offered to RA patients. In particular, two treatments with a similar efficacy profile may have different quality of life or physical function improvement profiles.

### SUMMARY

The inventors of the present invention have shown that a particular anti-IL6 receptor antibody, administered as a monotherapy, is capable of showing a remarkable efficacy for treating RA, and is also capable of remarkably improving the physical function and the quality of life of subjects suffering from RA.

Examples of embodiments of the invention are listed below:

### Embodiment 1

A method of improving the physical function of a subject suffering from rheumatoid arthritis, comprising administering to the subject an antibody, wherein:

- the antibody comprises the heavy chain variable region of sequence SEQ ID NO:1 and the light chain variable region of sequence SEQ ID NO:2,
- the antibody is administered subcutaneously at 150 mg or 200 mg once every two weeks,
- the subject is not administered with any other Disease-Modifying AntiRheumatic Drug (DMARD) in course of administration with the antibody,
- the subject was previously ineffectively treated for rheumatoid arthritis by administering at least one DMARD different from the antibody.

Embodiment 2

The method according to embodiment 1, wherein the subject achieves after at least 24 weeks of administration of the antibody a change from baseline (BL) in the Health Assessment Questionnaire Disability Index (HAQ-DI) of at least 0.22.

Embodiment 3

The method according to embodiment 1 or 2, wherein the subject achieves after at least 24 weeks of administration of the antibody a change from baseline (BL) in the Health Assessment Questionnaire Disability Index (HAQ-DI) of at least 0.30.

Embodiment 4

The method according to any one of embodiments 1-3, wherein the subject achieves after at least 24 weeks of administration of the antibody a change from baseline (BL) in the Health Assessment Questionnaire Disability Index (HAQ-DI) of at least 0.40.

Embodiment 5

The method according to any one of embodiments 1-4, wherein the subject achieves after at least 24 weeks of administration of the antibody a change from baseline (BL) in the Health Assessment Questionnaire Disability Index (HAQ-DI) of at least 0.50.

Embodiment 6

The method according to any one of embodiments 1-5, wherein the subject achieves after at least 24 weeks of administration of the antibody a change from baseline (BL) in the Health Assessment Questionnaire Disability Index (HAQ-DI) of at least 0.60.

Embodiment 7

The method according to any one of embodiments 1-6, wherein the subject achieves after at least 24 weeks of administration of the antibody a change from baseline (BL) in the Health Assessment Questionnaire Disability Index (HAQ-DI) of at least 0.61.

**Embodiment 8**

A method of improving the health related quality of life of a subject suffering from rheumatoid arthritis, comprising administering to the subject an antibody, wherein:

- the antibody comprises the heavy chain variable region of sequence SEQ ID NO:1 and the light chain variable region of sequence SEQ ID NO:2,
- the antibody is administered subcutaneously at 150 mg or 200 mg once every two weeks,
- the subject is not administered with any other Disease-Modifying AntiRheumatic Drug (DMARD) in course of administration with the antibody,
- the subject was previously ineffectively treated for rheumatoid arthritis by administering at least one DMARD different from the antibody.

Embodiment 9

The method according to embodiment 8, wherein the subject achieves after at least 24 weeks of administration of the antibody a change from baseline (BL) in the Short Form-36 Physical Component Summary (SF-36 PCS) of at least 2.5.

Embodiment 10

The method according to embodiment 8 or 9, wherein the subject achieves after at least 24 weeks of administration of the antibody a change from baseline (BL) in the Short Form-36 Physical Component Summary (SF-36 PCS) of at least 3.

## Embodiment 11

The method according to any one of embodiments 8-10, wherein the subject achieves after at least 24 weeks of administration of the antibody a change from baseline (BL) in the Short Form-36 Physical Component Summary (SF-36 PCS) of at least 4.

## Embodiment 12

The method according to any one of embodiments 8-11, wherein the subject achieves after at least 24 weeks of administration of the antibody a change from baseline (BL) in the Short Form-36 Physical Component Summary (SF-36 PCS) of at least 5.

## Embodiment 13

The method according to any one of embodiments 8-12, wherein the subject achieves after at least 24 weeks of administration of the antibody a change from baseline (BL) in the Short Form-36 Physical Component Summary (SF-36 PCS) of at least 6.

## Embodiment 14

The method according to any one of embodiments 8-13, wherein the subject achieves after at least 24 weeks of administration of the antibody a change from baseline (BL) in the Short Form-36 Physical Component Summary (SF-36 PCS) of at least 7.

## Embodiment 15

The method according to any one of embodiments 8-14, wherein the subject achieves after at least 24 weeks of administration of the antibody a change from baseline (BL) in the Short Form-36 Physical Component Summary (SF-36 PCS) of at least 8.

## Embodiment 16

The method according to any one of embodiments 8-15, wherein the subject achieves after at least 24 weeks of administration of the antibody a change from baseline (BL) in the Short Form-36 Physical Component Summary (SF-36 PCS) of 8.74.

## Embodiment 17

A method of treating rheumatoid arthritis in a subject, comprising administering to the subject an antibody, wherein:

- the antibody comprises the heavy chain variable region of sequence SEQ ID NO:1 and the light chain variable region of sequence SEQ ID NO:2,
- the antibody is administered subcutaneously at 150 mg or 200 mg once every two weeks,
- the subject is not administered with any other Disease-Modifying AntiRheumatic Drug (DMARD) in course of administration with the antibody,
- the subject was previously ineffectively treated for rheumatoid arthritis by administering at least one DMARD different from the antibody.

## Embodiment 18

The method according to embodiment 17, wherein the subject achieves after at least 24 weeks of administration of the antibody a 20% improvement in the American College of Rheumatology core set disease index (ACR20).

Embodiment 19

The method according to embodiment 17 or 18, wherein the subject achieves after at least 24 weeks of administration of the antibody a 50% improvement in the American College of Rheumatology core set disease index (ACR50).

Embodiment 20

The method according to any one of embodiments 17-19, wherein the subject achieves after at least 24 weeks of administration of the antibody a 70% improvement in the American College of Rheumatology core set disease index (ACR70).

Embodiment 21

The method according to any one of embodiments 17-20, wherein the subject achieves after at least 24 weeks of administration of the antibody a change from baseline (BL) in the Disease Activity Score 28 - Erythrocyte Sedimentation Rate (DAS28-ESR) of at least 2.

Embodiment 22

The method according to any one of embodiments 17-21, wherein the subject achieves after at least 24 weeks of administration of the antibody a change from baseline (BL) in the Disease Activity Score 28 - Erythrocyte Sedimentation Rate (DAS28-ESR) of at least 2.5.

Embodiment 23

The method according to any one of embodiments 17-22, wherein the subject achieves after at least 24 weeks of administration of the antibody a change from baseline (BL) in the Disease Activity Score 28 - Erythrocyte Sedimentation Rate (DAS28-ESR) of at least 3, particularly of at least 3.2, more particularly of 3.28.

Embodiment 24

The method according to any one of embodiments 17-23, wherein the subject achieves after at least 24 weeks of administration of the antibody a DAS28-ESR score below 3.2.

Embodiment 25

The method according to any one of embodiments 17-23, wherein the subject achieves after at least 24 weeks of administration of the antibody a DAS28-ESR score below 2.6.

Embodiment 26

The method according to any one of embodiments 1-25, wherein the subject who was previously ineffectively treated for rheumatoid arthritis by administering one or more DMARD different from the antibody, is a subject who had an inadequate response or intolerance to one or more Disease-Modifying Anti-Rheumatic Drugs (DMARDs).

Embodiment 27

The method according to any one of embodiments 1-26, wherein the subject who was previously ineffectively treated for rheumatoid arthritis by administering at least one DMARD different from the antibody, is a subject who was previously ineffectively treated for rheumatoid arthritis by administering Methotrexate.

Embodiment 28

The method according to any one of embodiments 1-27, wherein the subject who was previously ineffectively treated for rheumatoid arthritis by administering one or more DMARD different from the antibody, is a subject who had an inadequate response or intolerance to Methotrexate.

Embodiment 29

The method according to any one embodiments 1-28, wherein the subject suffering from rheumatoid arthritis is a subject suffering from moderately to severely active rheumatoid arthritis.

Embodiment 30

The method according to any one of embodiments 1-29, wherein the antibody is administered with a prefilled syringe.

Embodiment 31

The method according to any one of embodiments 1-30, wherein the antibody is administered with an auto-injector.

Embodiment 32

The method according to any one of embodiments 1-31, wherein the antibody is administered as an aqueous buffered solution at pH 6.0 containing 21 mM histidine, 45 mM arginine, 0.2% (w/v) polysorbate 20, 5% (w/v) sucrose.

Embodiment 33

The method according to embodiment 32, wherein the solution comprises at least 130 mg/mL of the antibody.

Embodiment 34

The method according to embodiment 32, wherein the solution comprises 131.6 mg/mL of the antibody.

Embodiment 35

The method according to embodiment 32, wherein the solution comprises 175 mg/mL of the antibody.

Embodiment 36

The method according to any one of embodiments 1-35, wherein the antibody comprising the heavy chain variable region of sequence SEQ ID NO:1 and the light chain variable region of sequence SEQ ID NO:2 is sarilumab.

**Embodiment 37**

An antibody for use in a method of improving the physical function of a subject suffering from rheumatoid arthritis, wherein:

- the antibody comprises the heavy chain variable region of sequence SEQ ID NO:1 and the light chain variable region of sequence SEQ ID NO:2,
- the antibody is administered subcutaneously at 150 mg or 200 mg once every two weeks to the subject,
- the subject is not administered with any other Disease-Modifying AntiRheumatic Drug (DMARD) in course of administration with the antibody,
- the subject was previously ineffectively treated for rheumatoid arthritis by administering at least one DMARD different from the antibody.

Embodiment 38

The antibody for use according to embodiment 37, wherein the subject achieves after at least 24 weeks of administration of the antibody a change from baseline (BL) in the Health Assessment Questionnaire Disability Index (HAQ-DI) of at least 0.22.

Embodiment 39

The antibody for use according to embodiment 37 or 38, wherein the subject achieves after at least 24 weeks of administration of the antibody a change from baseline (BL) in the Health Assessment Questionnaire Disability Index (HAQ-DI) of at least 0.30.

Embodiment 40

The antibody for use according to any one of embodiments 37-39, wherein the subject achieves after at least 24 weeks of administration of the antibody a change from baseline (BL) in the Health Assessment Questionnaire Disability Index (HAQ-DI) of at least 0.40.

Embodiment 41

The antibody for use according to any one of embodiments 37-40, wherein the subject achieves after at least 24 weeks of administration of the antibody a change from baseline (BL) in the Health Assessment Questionnaire Disability Index (HAQ-DI) of at least 0.50.

Embodiment 42

The antibody for use according to any one of embodiments 37-41, wherein the subject achieves after at least 24 weeks of administration of the antibody a change from baseline (BL) in the Health Assessment Questionnaire Disability Index (HAQ-DI) of at least 0.60.

Embodiment 43

The antibody for use according to any one of embodiments 37-42, wherein the subject achieves after at least 24 weeks of administration of the antibody a change from baseline (BL) in the Health Assessment Questionnaire Disability Index (HAQ-DI) of at least 0.61.

**Embodiment 44**

An antibody for use in a method of improving the health related quality of life of a subject suffering from rheumatoid arthritis, wherein:

- the antibody comprises the heavy chain variable region of sequence SEQ ID NO:1 and the light chain variable region of sequence SEQ ID NO:2,
- the antibody is administered subcutaneously at 150 mg or 200 mg once every two weeks to the subject,
- the subject is not administered with any other Disease-Modifying AntiRheumatic Drug (DMARD) in course of administration with the antibody,
- the subject was previously ineffectively treated for rheumatoid arthritis by administering at least one DMARD different from the antibody.

Embodiment 45

The antibody for use according to embodiment 44, wherein the subject achieves after at least 24 weeks of administration of the antibody a change from baseline (BL) in the Short Form-36 Physical Component Summary (SF-36 PCS) of at least 2.5.

Embodiment 46

The antibody for use according to embodiment 44 or 45, wherein the subject achieves after at least 24 weeks of administration of the antibody a change from baseline (BL) in the Short Form-36 Physical Component Summary (SF-36 PCS) of at least 3.

Embodiment 47

The antibody for use according to any one of embodiments 44-46, wherein the subject achieves after at least 24 weeks of administration of the antibody a change from baseline (BL) in the Short Form-36 Physical Component Summary (SF-36 PCS) of at least 4.

Embodiment 48

The antibody for use according to any one of embodiments 44-47, wherein the subject achieves after at least 24 weeks of administration of the antibody a change from baseline (BL) in the Short Form-36 Physical Component Summary (SF-36 PCS) of at least 5.

Embodiment 49

The antibody for use according to any one of embodiments 44-48, wherein the subject achieves after at least 24 weeks of administration of the antibody a change from baseline (BL) in the Short Form-36 Physical Component Summary (SF-36 PCS) of at least 6.

Embodiment 50

The antibody for use according to any one of embodiments 44-49, wherein the subject achieves after at least 24 weeks of administration of the antibody a change from baseline (BL) in the Short Form-36 Physical Component Summary (SF-36 PCS) of at least 7.

Embodiment 51

The antibody for use according to any one of embodiments 44-50, wherein the subject achieves after at least 24 weeks of administration of the antibody a change from baseline (BL) in the Short Form-36 Physical Component Summary (SF-36 PCS) of at least 8.

Embodiment 52

The antibody for use according to any one of embodiments 44-51, wherein the subject achieves after at least 24 weeks of administration of the antibody a change from baseline (BL) in the Short Form-36 Physical Component Summary (SF-36 PCS) of 8.74.

**Embodiment 53**

An antibody for use in a method of treating rheumatoid arthritis in a subject, wherein:

-   the antibody comprises the heavy chain variable region of sequence SEQ ID NO:1 and the light chain variable region of sequence SEQ ID NO:2,
-   the antibody is administered subcutaneously at 150 mg or 200 mg once every two weeks to the subject,
-   the subject is not administered with any other Disease-Modifying AntiRheumatic Drug (DMARD) in course of administration with the antibody,
-   the subject was previously ineffectively treated for rheumatoid arthritis by administering at least one DMARD different from the antibody.

Embodiment 54

The antibody for use according to embodiment 53, wherein the subject achieves after at least 24 weeks of administration of the antibody a 20% improvement in the American College of Rheumatology core set disease index (ACR20).

Embodiment 55

The antibody for use according to embodiment 53 or 54, wherein the subject achieves after at least 24 weeks of administration of the antibody a 50% improvement in the American College of Rheumatology core set disease index (ACR50).

Embodiment 56

The antibody for use according to any one of embodiments 53-55, wherein the subject achieves after at least 24 weeks of administration of the antibody a 70% improvement in the American College of Rheumatology core set disease index (ACR70).

Embodiment 57

The antibody for use according to any one of embodiments 53-56, wherein the subject achieves after at least 24 weeks of administration of the antibody a change from baseline (BL) in the Disease Activity Score 28 - Erythrocyte Sedimentation Rate (DAS28-ESR) of at least 2.

Embodiment 58

The antibody for use according to any one of embodiments 53-57, wherein the subject achieves after at least 24 weeks of administration of the antibody a change from baseline (BL) in the Disease Activity Score 28 - Erythrocyte Sedimentation Rate (DAS28-ESR) of at least 2.5.

Embodiment 59

The antibody for use according to any one of embodiments 53-58, wherein the subject achieves after at least 24 weeks of administration of the antibody a change from baseline (BL) in the Disease Activity Score 28 - Erythrocyte Sedimentation Rate (DAS28-ESR) of at least 3, particularly of at least 3.2, more particularly of 3.28

Embodiment 60

The antibody for use according to any one of embodiments 53-59, wherein the subject achieves after at least 24 weeks of administration of the antibody a DAS28-ESR score below 3.2.

Embodiment 61

The antibody for use according to any one of embodiments 53-60, wherein the subject achieves after at least 24 weeks of administration of the antibody a DAS28-ESR score below 2.6.

Embodiment 62

The antibody for use according to any one of embodiments 37-61, wherein the subject who was previously ineffectively treated for rheumatoid arthritis by administering one or more DMARD different from the antibody, is a subject who had an inadequate response or intolerance to one or more Disease-Modifying Anti-Rheumatic Drugs (DMARDs).

Embodiment 63

The antibody for use according to any one of embodiments 37-62, wherein the subject who was previously ineffectively treated for rheumatoid arthritis by administering at least one DMARD different from the antibody, is a subject who was previously ineffectively treated for rheumatoid arthritis by administering Methotrexate.

Embodiment 64

The antibody for use according to any one of embodiments 37-63, wherein the subject who was previously ineffectively treated for rheumatoid arthritis by administering one or more DMARD different from the antibody, is a subject who had an inadequate response or intolerance to Methotrexate.

Embodiment 65

The antibody for use according to any one embodiments 37-64, wherein the subject suffering from rheumatoid arthritis is a subject suffering from moderately to severely active rheumatoid arthritis.

Embodiment 66

The antibody for use according to any one of embodiments 37-65, wherein the antibody is administered with a prefilled syringe.

Embodiment 67

The antibody for use according to any one of embodiments 37-66, wherein the antibody is administered with an auto-injector.

Embodiment 68

The antibody for use according to any one of embodiments 37-67, wherein the antibody is administered as an aqueous buffered solution at pH 6.0 containing 21 mM histidine, 45 mM arginine, 0.2% (w/v) polysorbate 20, 5% (w/v) sucrose.

Embodiment 69

The antibody for use according to embodiment 68, wherein the solution comprises at least 130 mg/mL of the antibody.

Embodiment 70

The antibody for use according to embodiment 68, wherein the solution comprises 131.6 mg/mL of the antibody.

Embodiment 71

The antibody for use according to embodiment 68, wherein the solution comprises 175 mg/mL of the antibody.

Embodiment 72

The antibody for use according to any one of embodiments 37-71, wherein the antibody comprising the heavy chain variable region of sequence SEQ ID NO:1 and the light chain variable region of sequence SEQ ID NO:2 is sarilumab.

Embodiment 73

The antibody for use according to any one of embodiments 37-72, wherein the antibody is administered subcutaneously at 150 mg once every two weeks to the subject.

Embodiment 74

The antibody for use according to any one of embodiments 37-72, wherein the antibody is administered subcutaneously at 200 mg once every two weeks to the subject.

## DETAILED DESCRIPTION

It has been shown by the inventors that a particular anti-IL6R antibody administered as a monotherapy, is efficient for treating rheumatoid arthritis. In addition, the inventors have shown that the antibody administered as a monotherapy is also effective for improving the physical function and the Quality of Life of subjects suffering from rheumatoid arthritis.

According to the invention, "monotherapy" means that the subject receiving the antibody, is not administered with any other Disease-Modifying AntiRheumatic Drug (DMARD) in course of administration with the antibody,

The efficacy of the antibody for treating rheumatoid arthritis is typically measured using the standard methods in the field, commonly used by the clinicians and the rheumatologists, namely the DAS-28 and ACR parameters, particularly the DAS-28 ESR, ACR20, ACR50 and ACR70 parameters.

The improvement of the physical function of the subjects suffering from rheumatoid arthritis is typically measured using the standard methods in the field, commonly used by the clinicians and the rheumatologists, namely the HAQ-DI parameter.

The improvement of the quality of life of the subjects suffering from rheumatoid arthritis is typically measured using the standard methods in the field, commonly used by the clinicians and the rheumatologists, namely the SF36 parameter, more particularly the SF-36 PCS parameter.

Baseline is defined as the score obtained by the subject before being administered with the antibody according to the invention.

Change from baseline is defined as the difference existing between the score obtained by the subject at baseline and the score obtained by the subject after being administered with the antibody according to the invention, typically measured at least 24 weeks after the first administration of the antibody, particularly at 24 weeks after the first administration of the antibody.

**DAS28-ESR** (Disease Activity Score 28- Erythrocyte Sedimentation Rate)

DAS28 is a composite score that includes 4 variables:

- Tender Joints Count (based on 28 joints: shoulder (n=2), elbow (n=2), wrist (n=2), metacarpophalageal (n=10), interphalangeal of thumb (n=2), proximal interphalangeal (n=8), knee (n=2))
- Swollen Joints count (based on 28 joints: shoulder (n=2), elbow (n=2), wrist (n=2), metacarpophalageal (n=10), interphalangeal of thumb (n=2), proximal interphalangeal (n=8), knee (n=2))
- General health assessment (GH) by the patient assessed from the ACR RA core set questionnaire (patient global assessment) in 100 mm visual analogue scale (VAS)
- Marker of inflammation assessed by the CRP in mg/L or ESR in mm/hr.

It is a continuous measure allowing for measurement of absolute change in disease activity and percentage improvement. The DAS28-ESR can be calculated using the following formula:

$$\text{DAS28-ESR} = 0.56 \text{ x } \sqrt{28TJC} + 0.28 \text{ x } \sqrt{28SJC} + 0.70 \text{ x } \text{Ln}[\text{ESR(mm/h)}] + 0.014 \text{ x } \text{GH(VAS)}$$

The DAS28-ESR score provides a number indicating the current disease activity of the RA. A DAS28-ESR score above 5.1 means high disease activity, whereas a DAS28-ESR score below 3.2 indicates low disease activity and a DAS28-ESR score below 2.6 means disease remission.

When calculating the 28TJC and 28SJC, with individual missing joint scores (the 'replaced or fused' joints are not taken into consideration for the swelling or tenderness) imputed as the mean of the scored joints, the tender/swollen joint counts after imputation are as follows:

28TJC/28SJC = sum (scored tender/swollen joints)*(number of joints in the full joint set / number of scored tender/swollen joints). The number of joints in the full joint set is defined as (28 - number of replaced or fused joints) and the scored joints refer to those with an answer (0 - no pain, 1- pain).

If ESR=0, then ESR=1 for the purpose of the DAS28-ESR calculation to enable a non-missing score.

DAS28-ESR is considered as missing if one of the components is missing.

**ACR20**

To be classified as an ACR20 responder, a patient must achieve 20% improvement compared with baseline, in both TJC and SJC, as well as 20% improvement in at least 3 out of the 5 remaining ACR components: physician's global assessment of disease activity, patient's global assessment of disease activity, pain, HAQ-DI, and CRP.

**ACR50**

ACR50 is defined as the event of achieving at least 50% improvement in both TJC and SJC, and at least 50% improvement in at least 3 out of the 5 remaining ACR components.

**ACR70**

ACR70 is defined as the event of achieving at least 70% improvement in both TJC and SJC and at least 70% improvement in at least 3 out of the 5 remaining ACR components.

The 7 ACR components assessing the signs and symptoms of RA are defined below (A-G):

A) Tender Joint Count (TJC)

A total of 68 joints is assessed for tenderness. The 68 joints to be examined for tenderness are: temporomandibular (n=2), sternoclavicular (n=2), acromioclavicular (n=2), shoulder (n=2), elbow (n=2), wrist (n=2), metacarpophalageal (n=10), interphalangeal of thumb (n=2), distal interphalangeal (n=8), proximal interphalangeal (n=8), hip (n=2), knee (n=2), ankle mortise (n=2), ankle tarsus (n=2), metatarsophalangeal (n=10), interphalangeal of great toe (n=2), and proximal/distal interphalangeal of the toes (n=8).

A formal count of the joints is performed by a trained assessor. Joint tenderness is defined as pain induced by the pressure of the joints, exerted by the assessor's thumb and index finger. The assessor classifies each joint as painful yes/no and swollen yes/no. A score of 0/1 is given to each tender joint with 0 representing no pain and 1 representing pain. The tender joint count ranges from 0 to 68 where 0 is considered the best and 68 the worst.

B) Swollen Joint Count (SJC)

The 66 joints to be examined for swelling are the same as those examined for tenderness, except the hip joints are not included. A formal count of the joints is performed by a trained assessor. The assessor classifies each joint as swollen yes/no. A score of 0/1 is given to each swollen joint with 0 representing no swollen and 1 representing a swollen joint. The swollen joint count ranges from 0 to 66 where 0 is considered the best and 66 the worst.

C) Physician's Global Assessments of Disease Activity

Physician's global assessments of the patient's current disease activity is assessed on an anchored 100 mm horizontal VAS where 0 is considered the best disease activity (no disease activity) and 100 the worst (most disease activity).

D) Patient's Global Assessments of Disease Activity

Patient's global assessments of their current disease activity is rated on an anchored 100 mm horizontal VAS where 0 is considered the best disease activity (no disease activity) and 100 the worst (most disease activity).

E) Patient's Assessment of Pain

Patients are requested to indicate their pain intensity due to their RA using a 100 mm horizontal VAS where 0 is considered "No pain" and 100 "the worst pain you can imagine".

F) Patient's Assessment of Physical function - Health Assessment Questionnaire Disease Index (HAQ-DI)

The HAQ-DI is a standardized questionnaire developed for use in RA. The HAQ-DI, with the past Week as the time frame, focuses on whether the respondent "is able to..." do the activity and covers 8 categories in 20 items: dressing and grooming, arising, eating, walking, hygiene, reach, grip and activities, for which there are at least 2 questions by category. The 4 responses for the HAQ-DI questions are graded as follows: without any difficulty=0; with some difficulty=1; with much difficulty=2; and unable to do=3. To calculate the Standard HAQ-DI Score (With Aids/Devices), there are 3 steps:

1. Sum the 8 category scores by using the highest sub-category score from each category. For example, in the category EATING there are 3 sub-category items. A patient responds with a 1, 2, and 0, respectively; the category score is 2.
2. Adjust for use of aids/devices and/or help from another person when indicated.

- Adjust the score for a category by increasing a 0 or a 1 to a 2.
- If a patient's highest score for that sub-category is a 2 it remains a 2, and if a 3, it remains a 3.
- The data entered at field "Other specify" is not used for score adjustment.

3. Divide the summed category scores by the number of categories answered (must be a minimum of 6) to obtain a HAQ-DI score of 0 to 3 (3=worst functioning).

A HAQ-DI score cannot be calculated validly when there are scores for less than 6 of the 8 categories. HAQ-DI scoring ranges between 0 and 3. A high HAQ-DI score has been found to be a strong predictor of morbidity and mortality in RA. A 0.22 unit difference is considered clinically meaningful.

G) The level of an acute phase reactant measured by CRP

High sensitivity CRP is assessed centrally. Since CRP levels are directly correlated with Interleukin 6 receptor activity, it is expected that active dose regimens has a dramatic lowering effect on CRP levels. Therefore, during the study, post-dosing CRP remains blinded to the investigators, the sponsor and the patients.

The ACR components are further described in Table 1.

Table 1 - ACR components

| ACR components | Range | Direction |
| --- | --- | --- |
| TJC | 0 to 68 | Lower is better |
| SJC | 0 to 66 | Lower is better |
| Pain VAS | 0 to 100 | Lower is better |
| Patient global VAS | 0 to 100 | Lower is better |
| Physician global VAS | 0 to 100 | Lower is better |
| HAQ-DI | 0 to 3 | Lower is better |
| CRP (mg/dL) | >0 | Lower is better |

TJC: Total Joint Counts, SJC: Swollen Joint Counts, VAS: Visual Analog Scale

**SF-36 V2**

The QualityMetric's SF-36v2® Health Survey is a multi-purpose, short-form health survey with 36 questions. It yields scores for eight domains (Physical Functioning, Role-Physical, Bodily pain, General health, Vitality, Social Functioning, Role-Emotional, and Mental Health, each scale is scored from 0 to 100 where higher scores indicate better health and well-being), as well as two summary measures of physical and mental health: the physical component summary (PCS) and mental component summary (MCS).

The scoring process is summarized below:

1. Enter item response data.
2. Recode item response values.
3. Determine health domain scale raw scores.
4. Transform health domain scale raw scores to 0 to 100 scores.
5. Transform health domain scale 0 to 100 to normal-based scores.
6. Score PCS and MCS measures.

The following table shows the construction and summary measures of the SF-36 scales:

**Table: SF-36 V2 measurement model**

| Summary Measures | Scales | Items |
| --- | --- | --- |
| Physical Component Summary (PCS)* | Physical Functioning (PF) | 3a, 3b, 3c, 3d, 3e, 3f, 3g, 3h, 3i, 3j |
| | Role-Physical (RP) | 4a, 4b, 4c, 4d |
| | Bodily Pain (BP) | 7,8 |
| | General Health (GH) | 1, 11a, 11b, 11c, 11d |
| Mental Component Summary (MCS)* | Vitality (VT) | 9a, 9e, 9g, 9i |
| | Social Functioning (SF) | 6, 10 |
| | Role-Emotional (RE) | 5a, 5b, 5c |
| | Mental Health (MH) | 9b, 9c, 9d, 9f, 9h |
| *MCS and PCS derived from the eight scales (Ware, JE. et al. 1994) | | |

The Abbreviated Item Content is as follows:

3a Vigorous activities, such as running, lifting heavy objects, or participating in strenuous sports
3b Moderate activities, such as moving a table, pushing a vacuum cleaner, bowling, or playing golf
3c Lifting or carrying groceries
3d Climbing several flights of stairs
3e Climbing one flight of stairs
3f Bending, kneeling, or stooping
3g Walking more than a mile
3h Walking several hundred yards
3i Walking one hundred yards
3j Bathing or dressing oneself
4a Cut down the amount of time one spent on work or other activities
4b Accomplished less than you would like
4c Limited in kind of work or other activities
4d Had difficulty performing work or other activities (e.g., it took extra effort)
7 Intensity of bodily pain
8 Extent pain interfered with normal work
1 Is your health: excellent, very good, good, fair, poor
11 a Seem to get sick a little easier than other people
11b As healthy as anybody I know
11c Expect my health to get worse
11d Health is excellent
9a Feel full of life
9e Have a lot of energy
9g Feel worn out
9i Feel tired
6 Extent health problems interfered with normal social activities
10 Frequency health problems interfered with social activities
5a Cut down the amount of time spent on work or other activities
5b Accomplished less than you would like
5c Did work or other activities less carefully than usual
9b Been very nervous
9c Felt so down in the dumps that nothing could cheer you up
9d Felt calm and peaceful
9f Felt downhearted and depressed
9h Been happy

The PCS and MCS summary measure scores are computed if at least 50% of the component scales are available. The scale scores are computed if at least 50% of items are available within the corresponding scale. The missing items are imputed by the mean of available items.

General Scoring information

Items and scales are scored in 3 steps:

- Step 1. Item recoding, for the 10 items that require recoding,
- Step 2. Computing scale scores by summing across items in the same scale (raw scale scores); and,
- Step 3. Transforming raw scale scores to a 0-100 scale (transformed scale).

Item recoding

- All 36 items should be checked for out-of-range values prior to assigning the final item value. All out-of-range values should be recoded as missing data.
- The following tables show the recoding of response choice.

How to treat missing data

A scale score is calculated if a respondent answered at least half of the items in a multi-item scale (or half plus one in the case of scales with an odd number of items).

The recommended algorithm substitutes a person-specific estimate for any missing item when the respondent answered at least 50 percent of the items in a scale. A psychometrically sound estimate is the average score, across completed items in the same scale, for that respondent. For example, if a respondent leaves one item in the 5-item mental Health scale blank, substitute the respondent's average score (across the 4 completed mental health items) for that one item. When estimating the respondent's average score, use the respondent's final item values

Computing raw scale scores

After item recoding, including handling of missing data, a raw score is computed for each scale. This score is a simple algebraic sum of responses for all items in that scale. If the respondent answered at least 50% of the items in a multi-items scale, the score can be calculated. If the respondent did not answer at least 50% of the items, the score for that scale should be set to missing.

Transformation of the scale scores

The next step involves transforming each raw score to a 0 to 100 scale using the following formula:

$$\text{Transformed scale} = [(\text{actual raw score} - \text{lowest possible raw score}) / \text{possible raw score range}] \times 100$$

This transformation converts the lowest and highest possible scores to zero and 100, respectively.

Table - SF-36 V2 raw scores of eight domains

| Scale | Lowest, Highest possible raw scores | raw score range |
|---|---|---|
| Physical Functioning | 10,30 | 20 |
| Role-Physical | 4,20 | 16 |
| Bodily pain | 2,12 | 10 |
| General health | 5,25 | 20 |
| Vitality | 4,20 | 16 |
| Social Functioning | 2,10 | 8 |
| Role-Emotional | 3,15 | 12 |
| Mental Health | 5,25 | 20 |

The score of each of the 36 items is collected in CRF (case report form). Then, a SAS (Statistical Analysis System) code (e.g. the one provided by QualityMetric) is used to calculate the eight scales, the two summary measure scores and the standardized summary scores.

The PCS and MCS summary measure scores are computed if at least 50% of the component scales are available. The scale scores are computed if at least 50% of items are available within the corresponding scale. The missing items are imputed by the mean of available items.

Change from baseline (BL) in SF-36 scores (physical component summary score and mental component summary score as well as the eight domains) is then analyzed.

SF-36 V2 Scoring

*General Scoring information:*

Items and scales are scored in 3 steps (as instructed by QualityMetric):

Step 1. Item recoding, for then 10 items that require recoding

Step 2. Computing scale scores by summing across items in the same scale (raw scale scores); and

Step 3. Transforming raw scale scores to a 0-100 scale (transformed scale)

Step 5: Compute Z-Scores

Step 6: Convert Z-score to Norm Based scores for domains

PCS: Compute aggregate PCS score using a specific weighted formula, convert this into a Norm based score

MCS: Compute aggregate MCS score using a specific weighted formula, convert this into a Norm based score

*Item recoding:*

All 36 items should be checked for out-of-range values prior to assigning the final item value. All out-of-range values should be recoded as missing data.

*How to treat missing data*

A scale score is calculated if a respondent answered at least half of the items in a multi-item scale (or half plus one in the case of scales with an odd number of items).

The recommended algorithm substitutes a person-specific estimate for any missing item when the respondent answered at least 50 percent of the items in a scale. A psychometrically sound estimate is the average score, across completed items in the same scale, for that respondent. For example, if a respondent leaves one item in the 5-item mental Health scale blank, substitute the respondent's average score (across the 4 completed mental health items) for that one item. When estimating the respondent's average score, use the respondent's final item values.

*Computing raw scale scores*

After item recoding, including handling of missing data, a raw score is computed for each scale. This score is a simple algebraic sum of responses for all items in that scale. If the respondent answered at least 50% of the items in a multi-items scale, the score can be calculated. If the respondent did not answer at least 50% of the items, the score for that scale should be set to missing.

*Transformation of the scale scores*

The next step involves transforming each raw score to a 0 to 100 scale using the following formula:

$$\text{Transformed scale} = [(\text{actual raw score} - \text{lowest possible raw score}) / \text{possible raw score range}] \times 100$$

This transformation converts the lowest and highest possible scores to zero and 100, respectively.

**The Antibody**

The invention relates to methods comprising administering to the subject an antibody which comprises the heavy chain variable region of sequence SEQ ID NO:1 and the light chain variable region of sequence SEQ ID NO:2.

The disclosure provides pharmaceutical compositions comprising such antibody, and methods of using these compositions.

The antibody which comprises the heavy chain variable region of sequence SEQ ID NO:1 and the light chain variable region of sequence SEQ ID NO:2 is an antibody that specifically binds human interleukin-6 receptor (hIL-6R), as disclosed in WO2007/143168.

In one embodiment, the antibody which comprises the heavy chain variable region of sequence SEQ ID NO:1 and the light chain variable region of sequence SEQ ID NO:2 is sarilumab.

**DMARDs**

Disease modifying antirheumatic drugs (DMARDs) are drugs defined by their use in rheumatoid arthritis to slow down disease progression.

DMARDs have been classified as synthetic (sDMARD) and biological (bDMARD). Synthetic DMARDs include non-exhaustively methotrexate, sulfasalazine, leflunomide, hydroxychloroquine. Biological DMARDs include non-exhaustively adalimumab, golimumab, etanercept, abatacept, infliximab, rituximab, tocilizumab.

**Methods of Administration and Formulations**

According to the invention, the antibody is typically administered to the subject at 150 mg or 200 mg once every two weeks. "Once every two weeks" has the same meaning as "q2w" or "once per two weeks", i.e. that the antibody is administered once in a two week period of time.

The antibody is administered to the subject in a formulation comprising suitable carriers, excipients, and other agents to provide improved transfer, delivery, tolerance, and the like, and suitable for a subcutaneous injection.

The injectable preparations may be prepared by methods publicly known. For example, injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, *etc.,* which may be used in combination with an appropriate solubilizing agent such as an alcohol (e.g., ethanol), a polyalcohol (e.g., propylene glycol, polyethylene glycol), a nonionic surfactant [e.g., polysorbate 20 or 80, HCO-50 (polyoxyethylene (50 mol) adduct of hydrogenated castor oil)], *etc.* As the oily medium, there are employed, e.g., sesame oil, soybean oil, *etc.,* which may be used in combination with a solubilizing agent such as benzyl benzoate, benzyl alcohol, *etc.* The injection thus prepared can be filled in an appropriate ampoule.

The antibody is typically formulated as taught in WO2011/085158.

In a particular embodiment, the antibody is administered as an aqueous buffered solution at pH 6.0 containing

- 21 mM histidine,
- 45 mM arginine,
- 0.2% (w/v) polysorbate 20,
- 5% (w/v) sucrose
- between 100 mg/mL and 200 mg/mL of the antibody.

In another embodiment, the antibody is administered as an aqueous buffered solution at pH 6.0 containing

- 21 mM histidine,
- 45 mM arginine,
- 0.2% (w/v) polysorbate 20,
- 5% (w/v) sucrose
- at least 130 mg/mL of the antibody.

In another embodiment, the antibody is administered as an aqueous buffered solution at pH 6.0 containing

- 21 mM histidine,
- 45 mM arginine,
- 0.2% (w/v) polysorbate 20,
- 5% (w/v) sucrose
- 131.6 mg/mL of the antibody.

In another embodiment, the antibody is administered as an aqueous buffered solution at pH 6.0 containing

- 21 mM histidine,
- 45 mM arginine,
- 0.2% (w/v) polysorbate 20,
- 5% (w/v) sucrose
- 175 mg/mL of the antibody.

The antibody according to the invention can be administered to the subject using any acceptable device or mechanism. For example, the administration can be accomplished using a syringe and needle or with a reusable pen and/or autoinjector delivery device. The methods of the present invention include the use of numerous reusable pen and/or autoinjector delivery devices to administer an antibody (or pharmaceutical formulation comprising the antibody). Examples of such devices include, but are not limited to AUTOPEN™ (Owen Mumford, Inc., Woodstock, UK), DISETRONIC™ pen (Disetronic Medical Systems, Bergdorf, Switzerland), HUMALOG MIX 75/25™ pen, HUMALOG™ pen, HUMALIN 70/30™ pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™, OPTIPEN STARLET™, and OPTICLIK™ (sanofi-aventis, Frankfurt, Germany), to name only a few. Examples of disposable pen and/or autoinjector delivery devices having applications in subcutaneous delivery of a pharmaceutical composition of the present invention include, but are not limited to, the SOLOSTAR™ pen (sanofi-aventis), the FLEXPEN™ (Novo Nordisk), and the KWIKPEN™ (Eli Lilly), the SURECLICK™ Autoinjector (Amgen, Thousand Oaks, CA), the PENLET™ (Haselmeier, Stuttgart, Germany), the EPIPEN (Dey, L.P.), the HUMIRA™ Pen (Abbott Labs, Abbott Park, IL), the DAI® Auto Injector (SHL Group) and any auto-injector featuring the PUSHCLICK™ technology (SHL Group), to name only a few.

In one embodiment, the antibody is administered with a prefilled syringe.

In another embodiment, the antibody is administered with a prefilled syringe containing a safety system. In a particular embodiment, the antibody is administered with a prefilled syringe containing an ÈRIS™ safety system (West Pharmaceutical Services Inc.).

In another embodiment, the antibody is administered with an auto-injector. In a particular embodiment, the antibody is administered with an auto-injector featuring the PUSHCLICK™ technology (SHL Group).

**Patient Population**

According to the invention, "subject" means a human subject or human patient.

The antibody according to the invention is administered to subjects previously ineffectively treated for rheumatoid arthritis by administering one or more DMARD different from the antibody.

According to the invention, a subject who is considered "ineffectively treated" by his or her physician is a subject who either has shown to be intolerant to the one or more DMARD tested by the physician, and/or a subject who has shown an inadequate response to the one or more DMARD tested by the physician, typically a subject who is still considered by the physician to present an active rheumatoid arthritis despite the previous one or more DMARD administered. The "Active rheumatoid arthritis" is typically defined as:

- at least 6 of 66 swollen joints and 8 of 68 tender joints, as counted by the physician in a typical quantitative swollen and tender joint count examination,
- High sensitivity C-reactive protein (hs-CRP) $\geq$8mg/L or ESR $\geq$28mm/H
- DAS28ESR > 5.1.

In one embodiment, the subject who was previously ineffectively treated for rheumatoid arthritis by administering at least one DMARD different from the antibody is a subject who was previously ineffectively treated for rheumatoid arthritis by administering Methotrexate.

In another embodiment, the subject who was previously ineffectively treated for rheumatoid arthritis by administering one or more DMARD different from the antibody is a subject who had an inadequate response or intolerance to Methotrexate.

According to the invention, for those subjects previously ineffectively treated for rheumatoid arthritis by administering one or more DMARD different from the antibody, the one or more DMARD is/are not administered anymore to the subject, and the antibody is administered alone, in monotherapy to the subject.

All the references cited in the present disclosure are incorporated by reference.

**EXAMPLE: A randomized, double-blind, parallel-group study assessing the efficacy and safety of sarilumab monotherapy versus adalimumab monotherapy in patients with rheumatoid arthritis (Study No. EFC14092, Study Title: SARIL-RA-MONARCH)**

**Objectives:**

Primary objective: To demonstrate that sarilumab monotherapy is superior to adalimumab monotherapy with respect to signs and symptoms as assessed by the Disease Activity Score 28 erythrocyte sedimentation rate (DAS28-ESR) at Week 24 in patients with active Rheumatoid arthritis (RA) who are either intolerant of, or considered inappropriate candidates for continued treatment with Methotrexate (MTX), or after at least 12 weeks of continuous treatment with MTX, are determined to be inadequate responders.

Secondary objectives: To demonstrate that sarilumab monotherapy is superior to adalimumab monotherapy in patients with active RA who are either intolerant of, or considered inappropriate candidates for continued treatment with MTX, or after at least 12 weeks of continuous treatment with MTX, are determined to be inadequate responders, with respect to:

- reduction of signs and symptoms of RA at Week 24
- improvement in quality of life as measured by patient reported outcomes questionnaires at Week 24

To assess the safety and tolerability of sarilumab monotherapy (including immunogenicity) throughout the study.

**Methodology:**

Randomized, double-blind, double-dummy, parallel-group study for 24 weeks followed by open-label sarilumab treatment. Randomization was stratified by regions.

**Number of patients:**

Planned: 340
Randomized: 369
Treated: 368

Evaluated:    Efficacy: 369
Safety: 368

**Diagnosis and criteria for inclusion:**

Patients with active RA for $\geq$ 3 months who were either intolerant of, or considered inappropriate candidates for continued treatment with MTX; or after at least 12 weeks of continuous treatment with MTX, are determined to be inadequate responders.

**Study treatments**

Investigational medicinal product(s): sarilumab 200 mg q2w or placebo and adalimumab 40 mg q2w or placebo in prefilled syringes for subcutaneous administration.
Duration of treatment: 24 Weeks of randomized treatment
Duration of observation: Up to 34 weeks for the randomized period (4 week screening, 24 weeks of treatment, and 6 week posttreatment observation if patient doesn't enter the open-label extension)

**Criteria for evaluation:**

Efficacy:

Primary endpoint: Change from baseline in DAS28-ESR at Week 24
Secondary endpoints:

DAS28-ESR (remission) - Week 24
ACR50 response - Week 24
ACR70 response - Week 24
ACR20 response - Week 24
HAQ-DI - Week 24
SF-36 Physical - Week 24
FACIT Fatigue - Week 24
SF-36 Mental - Week 24

Safety: Adverse events reported by the patient or noted by the Investigator, adjudicated cardiovascular events and standard hematology and blood chemistry, electrocardiogram (ECG), physical examinations, and occurrence of anti-drug antibodies to sarilumab.

Statistical methods:

The efficacy analysis population is the intent-to-treat (ITT) population, which includes all randomized subjects. For efficacy analysis, subjects were analyzed in the treatment group to which they were randomized, irrespective of the treatment they actually received. The primary safety analysis was conducted on all randomized patients who were exposed to at least one injection of the study medication. Subjects are analyzed in the treatment group that they actually received, irrespective of which group they were randomized to. For the primary efficacy, the change from baseline in the DAS28-ESR at Week 24, data collected on or before Week 24 including after adalimumab (or matching placebo) dose increase were used. Data collected after treatment discontinuation was set to missing. No imputation was performed. The primary efficacy endpoint was assessed by using a mixed model for repeated measures (MMRM) with the baseline covariate and factors for treatment, region, visit and visit by treatment interaction.

Safety summaries were descriptive and no hypothesis testing was conducted. Summary of treatment emergent adverse events (TEAE) was based on MedDRA coding of verbatim terms reported by investigators. TEAEs were defined as any adverse events that newly developed or worsened or became serious on or after the day of first dose intake of investigational medicinal product (IMP), up to the day of end of study or until start of extension treatment. For selected laboratory tests, vital signs and ECGs, incidences of potentially clinically significant abnormality (PCSA) values were summarized.

Population characteristics:

Three hundred sixty-nine (369) patients represented the ITT population. Three hundred sixty-eight (368) patients represented the safety population. 321 (87%) patients successfully completed the 24-week treatment period, of which 320 patients continued in the open-label extension period to continue long term treatment with sarilumab. 47 (12.7%) patients permanently discontinued the double-blind study treatment, of which 17 patients continued follow-up visit to Week 24. The demographic and disease characteristics at baseline were generally similar between treatment groups. Patients on sarilumab tended to be younger with a longer duration of RA and lower baseline C-reactive protein (CRP) compared to adalimumab.

The mean duration of study treatment was 158 days in the sarilumab group and 154 days in the adalimumab group. The percentage of patients with IMP compliance≥80% was 99 % and 100%, respectively.

**Efficacy Results:**

Primary endpoint

The change in the DAS28-ESR score from baseline to Week 24 showed a significantly greater decrease in the sarilumab group compared to adalimumab (with a mean difference of -1.077 units, p-value <0.0001). This effect was seen as early as Week 12. Both planned sensitivity analyses confirmed these results.

| | Adalimumab 40mg q2w (N=185) | Sarilumab 200mg q2w (N=184) |
|---|---|---|
| DAS28-CRP | | |
| Number | 156 | 163 |
| Baseline Mean (SD) | 5.98 (0.88) | 6.00 (0.87) |
| Week 24 Mean (SD) | 3.92 (1.24) | 3.07 (1.21) |
| Change Mean (SD) | -2.06 (1.22) | -2.93 (1.25) |
| LS mean (SE) | -1.97 (0.094) | -2.86 (0.093) |
| LS mean diff, 95% CI | | -0.884 (-1.138,-0.629) |
| P-value vs Adalimumab[a] | | <0.0001 |
| All assessments are set to missing from the time a patient prematurely discontinues study medication. | | |

Secondary endpoints

Table below shows the results for the pre-specified hierarchy of primary and secondary efficacy endpoints including assessments of quality of life / physical function. The results that are bolded are statistically significant according to the procedure of analysis. The last statistically significant endpoint in the testing hierarchy was the SF-36 physical score.

| Parameter | Adalimumab 40 mg q2w (N=185) | Sarilumab 200 mg q2w (N=184) | P-value | Delta |
|---|---|---|---|---|
| | Estimate | | P-value | Delta |
| **Primary endpoint** | | | | |
| **DAS28-ESR** | **-2.20(0.106)** | **-3.28(0.105)** | **<0.0001** | **-1.08** |
| **Secondary endpoints** | | | | |
| **DAS28-ESR remission (< 2.6) - Week 24** | **13(7.0%)** | **49(26.6%)** | **<0.0001** | **19.6%** |
| **ACR50 response - Week 24** | **55 (29.7%)** | **84 (45.7%)** | **0.0017** | **16.0%** |
| **ACR70 response - Week 24** | **22(11.9%)** | **43 (23.4%)** | **0.0036** | **11.5%** |
| **ACR20 response - Week 24** | **108(58.4%)** | **132(71.7%)** | **0.0074** | **13.3%** |
| **HAQ-DI - Week 24** | **-0.43(0.045)** | **-0.61(0.045)** | **0.0037** | **-0.18** |
| **SF-36 Physical - Week 24** | **6.09(0.555)** | **8.74(0.555)** | **0.0006** | **2.65** |
| FACIT Fatigue - Week 24 | 8.41(0.709) | 10.18(0.701) | 0.0689 | 1.77 |
| SF-36 Mental - Week 24 | 6.83(0.774) | 7.86(0.773) | 0.3319 | 1.03 |

More detailed analysis of the HAQ-DI scores are provided below.

| Change from baseline in HAQ-DI at Week 24 - ITT population | | |
|---|---|---|
| | **Adalimumab 40mg q2w (N=185)** | **Sarilumab 200mg q2w (N=184)** |
| HAQ-DI | | |
| Number | 158 | 165 |
| Baseline Mean (SD) | 1.62 (0.64) | 1.64 (0.54) |
| Week24 Mean (SD) | 1.21 (0.66) | 1.01 (0.65) |
| | | |
| Change Mean (SD) | -0.42 (0.58) | -0.63 (0.66) |
| LS mean (SE) | **-0.43** (0.045) | **-0.61** (0.045) |
| LS mean diff, 95% CI | | -0.182 (-0.305,-0.059) |
| P-value vs Adalimumab | | 0.0037 |

| HAQDI Responder (≥0.3) Week 24 n(%) | Adalimumab 40mg q2w (N=185) | Sarilumab 200mg q2w (N=184) |
|---|---|---|
| Number | 185 | 184 |
| Responders | 88 (47.6%) | 114 (62.0%) |
| Non-responders | 97 (52.4%) | 70 (38.0%) |
| P-value vs Adalimumab | | 0.0057 |
| OR, CI vs Adalimumab | | 1.785 (1.180, 2.698) |

| HAQDI Responder (≥0.22) Week 24 n(%) | Adalimumab 40mg q2w (N=185) | Sarilumab 200mg q2w (N=184) |
|---|---|---|
| Number | 185 | 184 |
| Responders | 100 (54.1%) | 124 (67.4%) |
| Non-responders | 85 (45.9%) | 60 (32.6%) |

(continued)

| HAQDI Responder (≥0.22) Week 24 n(%) | Adalimumab 40mg q2w (N=185) | Sarilumab 200mg q2w (N=184) |
|---|---|---|
| P-value vs Adalimumab | | 0.0090 |
| OR, CI vs Adalimumab | | 1.747 (1.147, 2.663) |

## Claims

1. An antibody for use in a method of improving the physical function of a subject suffering from rheumatoid arthritis, wherein:

   - the antibody comprises the heavy chain variable region of sequence SEQ ID NO:1 and the light chain variable region of sequence SEQ ID NO:2,
   - the antibody is administered subcutaneously at 150 mg or 200 mg once every two weeks to the subject,
   - the subject is not administered with any other Disease-Modifying AntiRheumatic Drug (DMARD) in course of administration with the antibody,
   - the subject was previously ineffectively treated for rheumatoid arthritis by administering at least one DMARD different from the antibody.

2. The antibody for use according to Claim 1, wherein the subject achieves after at least 24 weeks of administration of the antibody a change from baseline (BL) in the Health Assessment Questionnaire Disability Index (HAQ-DI) of at least 0.22, particularly of at least 0.30, more particularly of at least 0.60.

3. An antibody for use in a method of improving the health related quality of life of a subject suffering from rheumatoid arthritis, wherein:

   - the antibody comprises the heavy chain variable region of sequence SEQ ID NO:1 and the light chain variable region of sequence SEQ ID NO:2,
   - the antibody is administered subcutaneously at 150 mg or 200 mg once every two weeks to the subject,
   - the subject is not administered with any other Disease-Modifying AntiRheumatic Drug (DMARD) in course of administration with the antibody,
- the subject was previously ineffectively treated for rheumatoid arthritis by administering at least one DMARD different from the antibody.

4. The antibody for use according to Claim 3, wherein the subject achieves after at least 24 weeks of administration of the antibody a change from baseline (BL) in the Short Form-36 Physical Component Summary (SF-36 PCS) of at least 2.5, particularly of at least 3, more particularly of at least 8.

5. An antibody for use in a method of treating rheumatoid arthritis in a subject, wherein:

- the antibody comprises the heavy chain variable region of sequence SEQ ID NO:1 and the light chain variable region of sequence SEQ ID NO:2,
- the antibody is administered subcutaneously at 150 mg or 200 mg once every two weeks to the the subject,
- the subject is not administered with any other Disease-Modifying AntiRheumatic Drug (DMARD) in course of administration with the antibody,
- the subject was previously ineffectively treated for rheumatoid arthritis by administering at least one DMARD different from the antibody.

6. The antibody for use according to Claim 5, wherein the subject achieves after at least 24 weeks of administration of the antibody a 20% improvement in the American College of Rheumatology core set disease index (ACR20), particularly a 50% improvement in the American College of Rheumatology core set disease index (ACR50), more particularly a 70% improvement in the American College of Rheumatology core set disease index (ACR70).

7. The antibody for use according to any one of Claims 5-6, wherein the subject achieves after at least 24 weeks of administration of the antibody a change from baseline (BL) in the Disease Activity Score 28 - Erythrocyte Sedimentation Rate (DAS28-ESR) of at least 2, particularly of at least 2.5, more particularly of at least 3.

8. The antibody for use according to any one of Claims 5-7, wherein the subject achieves after at least 24 weeks of administration of the antibody a DAS28-ESR score below 3.2, particularly below 2.6.

9. The antibody for use according to any one of Claims 1-8, wherein the subject who was previously ineffectively treated for rheumatoid arthritis by administering at least one DMARD different from the antibody, is a subject who was previously ineffectively treated for rheumatoid arthritis by administering Methotrexate.

10. The antibody for use according to any one Claims 1-9, wherein the subject suffering from rheumatoid arthritis is a subject suffering from moderately to severely active rheumatoid arthritis.

11. The antibody for use according to any one of Claims 1-10, wherein the antibody is administered with a prefilled syringe or with an auto-injector.

12. The antibody for use according to any one of Claims 1-11, wherein the antibody is administered as an aqueous buffered solution at pH 6.0 containing 21 mM histidine, 45 mM arginine, 0.2% (w/v) polysorbate 20, 5% (w/v) sucrose.

13. The antibody for use according to Claim 12, wherein the solution comprises at least 130 mg/mL of the antibody, particularly the solution comprises 131.6 mg/mL of the antibody.

14. The antibody for use according to Claim 12, wherein the solution comprises 175 mg/mL of the antibody.

15. The antibody for use according to any one of Claims 1-14, wherein the antibody comprising the heavy chain variable region of sequence SEQ ID NO:1 and the light chain variable region of sequence SEQ ID NO:2 is sarilumab.

Europäisches
Patentamt

European
Patent Office

Office européen
des brevets

# EUROPEAN SEARCH REPORT

Application Number

EP 16 30 5253

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2013/053751 A1 (SANOFI SA [FR]; REGENERON PHARMA [US]) 18 April 2013 (2013-04-18) * page 26; claims 1,2 * | 1-15 | INV. A61K39/395 C07K16/28 A61K39/00 |
| A | WO 2015/077582 A1 (SANOFI BIOTECHNOLOGY [FR]; REGENERON PHARMA [US]) 28 May 2015 (2015-05-28) * claims 20-23 * | 1-15 | |
| A | US 2008/131374 A1 (MEDICH JOHN R [US] ET AL) 5 June 2008 (2008-06-05) | 1-15 | |

TECHNICAL FIELDS
SEARCHED      (IPC)

A61K
C07K

The present search report has been drawn up for all claims

| Place of search | Date of completion of the search | Examiner |
|---|---|---|
| Munich | 15 June 2016 | Turri, Matteo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 30 5253

This annex lists the patent family members relating to the patent documents cited in the above-mentioned European search report.
The members are as contained in the European Patent Office EDP file on
The European Patent Office is in no way liable for these particulars which are merely given for the purpose of information.

15-06-2016

| Patent document cited in search report | Publication date | Patent family member(s) | | Publication date |
|---|---|---|---|---|
| WO 2013053751 A1 | 18-04-2013 | AR | 088289 A1 | 21-05-2014 |
| | | AU | 2012323062 A1 | 01-05-2014 |
| | | CA | 2851751 A1 | 18-04-2013 |
| | | CN | 104105505 A | 15-10-2014 |
| | | EP | 2766039 A1 | 20-08-2014 |
| | | JP | 2014530226 A | 17-11-2014 |
| | | KR | 20140123474 A | 22-10-2014 |
| | | RU | 2014118741 A | 20-11-2015 |
| | | SG | 11201401373R A | 29-05-2014 |
| | | US | 2014302053 A1 | 09-10-2014 |
| | | UY | 34387 A | 31-05-2013 |
| | | WO | 2013053751 A1 | 18-04-2013 |
| WO 2015077582 A1 | 28-05-2015 | CA | 2930615 A1 | 28-05-2015 |
| | | WO | 2015077582 A1 | 28-05-2015 |
| US 2008131374 A1 | 05-06-2008 | US | 2008131374 A1 | 05-06-2008 |
| | | US | 2012171123 A1 | 05-07-2012 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2007143168 A

- WO 2011085158 A

**Non-patent literature cited in the description**

- *British Journal of Rheumatology,* 1997, vol. 36, 884-888